# EUROPÄISCHE PATENTSCHRIFT

(11) **EP 0 613 457 B1**
(45) Veröffentlichungstag und Bekanntmachung des Hinweises auf die Patenterteilung: **19.06.1996**
(21) Anmeldenummer: 92922862.5
(22) Anmeldetag: 04.11.1992
(51) Int. Cl.: C07C 69/30, C07C 69/33, C07C 69/52, C07C 67/08, C07C 41/03, C08G 65/32

(54) **ESTER VON FETTSÄUREN MIT ETHOXYLIERTEN POLYOLEN**
ESTERS OF FATTY ACIDS WITH ETHOXYLATED POLYOLS
ESTERS D'ACIDES GRAS AVEC POLYOLS ETHOXYLES

(30) Priorität: 13.11.1991 DE 4137317
(43) Veröffentlichungstag der Anmeldung: 07.09.1994
(73) Patentinhaber: Henkel Kommanditgesellschaft auf Aktien, 40191 Düsseldorf (DE)
(72) Erfinder: TRIUS OLIVA, Antonio, E-Valldoreix (sant cugat) (ES); PONSATI OBIOLS, Oriol, E-08025 Barcelona (ES); BIGORRA LLOSAS, Joaquim Callassanc Duran, E-08203 Sabadell (ES); PRAT QUERALT, Esther, E-08328 Alella (ES)
(86) Internationale Anmeldenummer: EP9202525
(87) Internationale Veröffentlichungsnummer: WO9310072

(56) Entgegenhaltungen:
- EP-A- 0 356 255
- WO-A-90/05714
- FR-A- 2 534 923

## Beschreibung

### Gebiet der Erfindung

Die Erfindung betrifft Ester von Fettsäuren mit ethoxylierten Polyolen, ein Verfahren zur ihrer Herstellung sowie ihre Verwendung als Verdickungsmittel für wäßrige Tensidlösungen.

### Stand der Technik

Wäßrige Tensidlösungen, insbesondere solche, die im Bereich der Körperpflege als Haarshampoos, Schaumbäder, Duschbäder, Handwaschpasten und dergleichen zum Einsatz gelangen, enthalten als Tensidkomponenten in der Regel anionische oder amphotere Tenside, beispielsweise vom Typ der Fettalkoholethersulfate, Alkansulfonate, Sulfosuccinate, Ethercarbonsäuren oder Betaine. Um diese klaren oder dispersen Systeme zu stabilisieren und ihr Fließverhalten so zu verbessern, daß ein einfaches Dosieren möglich wird, werden den Tensidlösungen üblicherweise Verdickungsmittel zugesetzt [**Seifen-Öle- Fette-Wachse, 116, 60 (1990)**].

Dem Fachmann sind für diesen Zweck bereits eine Vielzahl von anorganischen und organischen Stoffen bekannt, die zur Erhöhung der Viskosität wäßriger Tensidlösungen geeignet sind.

Als anorganische Verdickungsmittel kommen eine Reihe wasserlöslicher Salze, beispielsweise Kochsalz, in Betracht [**Seifen-Öle-Fette-Wachse, 113, 135 (1987)**].

Unter den organischen Verdickungsmitteln sind beispielsweise Polyethylenglykoldifettsäureester [**DE 35 41 813 A1, DE 35 51 535 A1. DE 36 00 263 A1**], Glycerintrifettsäureester [**Cosm. Toil., 103, 99 (1988)**], Anlagerungsprodukte von durchschnittlich 50-60 Mol Ethylenoxid an Glycerinester (FR-A 2534923), ethoxylierte Fettalkohole [**DE 37 30 179 A1, EP 0343 463 A2**], wasserlösliche Polymere sowie Fettsäurealkanolamide bekannt.

In den meisten Fällen ist es nicht oder nur bei hohen Konzentrationen möglich, allein durch Verwendung anionischer Salze die gewünschte Viskosität in der Tensidlösung aufzubauen. Man ist daher in der Regel gezwungen, zusätzlich zu den anorganischen Salzen organische Verdickungsmittel einzusetzen, die mit einer Reihe von Nachteilen verbunden sind. So weisen die mit Polyethylenglycolfettsäurediestern, Glycerintrifettsäureestern oder Fettalkoholethoxylaten verdickten Lösungen häufig eine unzureichende Viskositätsstabilität bei Lagerung auf, während wasserlösliche Polymere sich oft nur schwer auflösen und ein unerwünschtes, schleimiges Fließverhalten mit der Neigung, Fäden zu ziehen, an den Tag legen.

Wegen ihrer ausgezeichneten Verdickungswirkung haben die Fettsäurealkanolamide lange Zeit eine besondere Bedeutung besessen. Infolge eines geringen, herstellungsbedingten Gehaltes an freien Alkanolaminen, der Anlaß für die Bildung von Nitrosaminen sein kann, kommen diese Stoffe für die Verwendung in kosmetischen Produkten nur noch in begrenztem Umfang in Betracht. Dies gilt im übrigen auch für praktisch alle übrigen stickstoffhaltigen Verdickungsmittel.

Die Aufgabe der Erfindung bestand somit darin, neue Verdikkungsmittel für wäßrige Tensidlösungen zu entwickeln, die frei von den geschilderten Nachteilen sind.

### Beschreibung der Erfindung

Gegenstand der Erfindung sind Ester von Fettsäuren mit ethoxylierten Polyolen, die erhalten werden, indem man
a) Polyole in Gegenwart basischer Katalysatoren bei erhöhten Temperaturen mit 110 bis 200 mol Ethylenoxid pro mol Polyol ethoxyliert und
b) das Reaktionsprodukt anschließend in Gegenwart saurer Katalysatoren mit 1 bis 1,3 mol Fettsäure pro mol der im ursprünglichen Polyol enthaltenen Hydroxylgruppen umsetzt.

Überraschenderweise wurde gefunden, daß sich die erfindungsgemäßen Ester als besonders wirksame stickstofffreie Verdikkungsmittel für wäßrige Tensidlösungen eignen.

Ester mit besonders vorteilhaften verdickenden Eigenschaften leiten sich von Addukten von 90 bis 150 mol Ethylenoxid an ein mol Glycerin ab, die mit gesättigten Fettsäuren mit 12 bis 18 Kohlenstoffatomen verestert worden sind.

Ein weiterer Gegenstand des Patentes betrifft ein Verfahren zur Herstellung von Estern von Fettsäuren mit ethoxylierten Polyolen, das sich dadurch auszeichnet, daß man
a) Polyole in Gegenwart basischer Katalysatoren bei erhöhten Temperaturen mit 110 bis 200 mol Ethylenoxid pro mol Polyol ethoxyliert und
b) das Reaktionsprodukt anschließend in Gegenwart saurer Katalysatoren mit 1 bis 1,3 mol Fettsäure pro mol der im ursprünglichen Polyol enthaltenen Hydroxylgruppen umsetzt.

Unter **Polyole**, die als Ausgangsmaterialien für die erfindungsgemäßen Ester in Betracht kommen, sind solche Stoffe zu verstehen, die über mindestens drei freie Hydroxylgruppen verfügen. Typische Beispiele sind Glycerin, Diglycerin, Triglycerin, Oligoglycerine mit einem durchschnittlichen Kondensationsgrad von 4 bis 10, Trimethylolpropan und Pentaerythrit. Bevorzugt ist der Einsatz von Glycerin.

Die Ethoxylierung der Polyole kann in an sich bekannter Weise durchgeführt werden. Als **basische Katalysatoren** kommen dabei beispielsweise Natriumhydroxid, Kaliumhydroxid, Natriummethylat, calcinierter Hydrotalcit [**DE 38 43 713 A1**] und mit Fettsäuren hydrophobierter Hydrotalcit [**DE 40 10 606 A1**] in Betracht.

Das molare Einsatzverhältnis von Polyol zu Ethylenoxid kann 1 : 110 bis 1 : 200 betragen. Im Hinblick auf Ester mit besonders vorteilhaften verdickenden Eigenschaften hat es sich als optimal erwiesen, ein Verhältnis von 1 : 110 bis 1 : 150 zu wählen.

Die Ethoxylierung wird gemäß den Bedingungen des Stands der Technik bei erhöhten Temperaturen, beispielsweise 120 bis 190 und vorzugsweise 150 bis 180°C und Drücken von 1 bis 5 bar durchgeführt. Die Anlagerung des Ethylenoxids an die freien Hydroxylgruppen der Polyole folgt dabei den Gesetzen der Statistik.

Im Anschluß an die Ethoxylierung werden die als Zwischenprodukte gebildeten ethoxylierten Polyole einer Veresterung unterworfen. Als Carbonsäurekomponente kommen hierzu **Fettsäuren** der Formel (I) in Betracht,

R¹CO-OH (I)

in der R¹CO für einen linearen oder verzweigten, aliphatischen, gegebenenfalls hydroxysubstituierten Acylrest mit 6 bis 22 Kohlenstoffatomen und 0, 1, 2 oder 3 Doppelbindungen steht.

Typische Beispiele sind Capronsäure, Caprylsäure, Caprinsäure, Laurinsäure, Myristinsäure, Palmitinsäure, Palmitoleinsäure, Stearinsäure, Ölsäure, Elaidinsäure, Petroselinsäure, Linolsäure, Linolensäure, Ricinolsäure, 12-Hydroxystearinsäure, Arachinsäure, Gadoleinsäure, Behensäure und Erucasäure. Bevorzugt sind Palmitin- und Stearinsäure sowie deren technische Gemische.

Wie in der Fettchemie üblich, können diese Säuren auch als technische Schnitte vorliegen, wie sie bei der Druckspaltung von natürlichen Fetten und Ölen, beispielsweise Palmöl, Palmkernöl, Kokosöl, Olivenöl, Sonnenblumenöl, Rüböl oder Rindertalg anfallen. Bevorzugt sind gesättigte Fettsäuren mit 12 bis 18, insbesondere 16 bis 18 Kohlenstoffatomen.

Die Veresterung der als Zwischenprodukte gebildeten ethoxylierten Polyole mit den Fettsäuren kann ebenfalls in an sich bekannter Weise durchgeführt werden. Als **saure Katalysatoren** kommen hierfür beispielsweise Methansulfonsäure, Butansulfonsäure, p-Toluolsulfonsäure, Naphthalinsulfonsäure, Alkylbenzolsulfonsäure und/oder Sulfobernsteinsäure in Betracht.

Es empfiehlt sich ferner, die Veresterung bei erhöhten Temperaturen, beispielsweise bei 140 bis 200, vorzugsweise bei 150 bis 170°C durchzuführen und das Reaktionswasser kontinuierlich aus dem Gleichgewicht zu entfernen. Die Einsatzmenge an Fettsäure ist dabei so auszuwählen, daß auf jedes mol der im ursprünglichen Polyol enthaltenen Hydroxylgruppen 1,0 bis 1,3, vorzugsweise 1,0 bis 1,15 mol Fettsäure entfallen. Auf diese Weise ist sichergestellt, daß die Veresterung der Hydroxylgruppen praktisch quantitativ ist. Ein Restgehalt freier Fettsäure im Reaktionsendprodukt kann - falls dies gewünscht wird - mit Alkalihydroxidlösung neutralisiert werden.

Bei Zusatz der erfindungsgemäßen Ester zu wäßrigen Tensidlösungen ist eine verdickende Wirkung zu beobachten, die durch Zusatz von anorganischen Elektrolytsalzen noch gesteigert werden kann.

Ein weiterer Gegenstand der Erfindung betrifft daher die Verwendung der erfindungsgemäßen Ester als Verdickungsmittel für wäßrige Tensidlösungen, denen sie in Mengen von 0,1 bis 5, vorzusgweise 1 bis 3 Gew.-% - bezogen auf den Feststoffgehalt der Lösungen - zugesetzt werden können.

Unter **Tensiden**, die im Sinne der Erfindung vorteilhaft verdickt werden können, sind beispielsweise Fettalkoholsulfate, Fettalkoholethersulfate, Alkansulfonate, Sulfobernsteinsäuremono- und -diester, Alkylphosphate, Eiweißfettsäurekondensate, Acylisethionate, Acyltauride, Seifen, Alkylethercarbonsäuren, Acylsarcoside, Alkylamidobetaine, Imidazoliniumbetaine oder Sulfobetaine zu verstehen, die einen lipophilen Fettrest mit 6 bis 22 Kohlenstoffatomen aufweisen. Tenside, deren wäßrige Lösungen sich durch den Zusatz der erfindungsgemäßen Ester besonders gut verdicken lassen, sind einerseits Fettalkoholethersulfate, die 12 bis 18 Kohlenstoffatome im Fettalkylrest und 1 bis 10 Ethylenoxideinheiten in der Polyetherkette aufweisen sowie Betaine. Im Hinblick auf Konstitution und Herstellung dieser Tenside sei auf **J.Falbe (ed.), "Surfactants in Consumer Products", Springer Verlag, Berlin, 1986, S.54 - 85** verwiesen.

Die Tensidlösungen können einen Feststoffgehalt von 1 bis 50, vorzugsweise 5 bis 25 Gew.-% - bezogen auf die Lösung - aufweisen.

In untergeordneten Mengen können ferner Duftstoffe, Farbstoffe, Trübungs- und Perlglanzmittel, antimikrobielle Stoffe, Konservierungsmittel, hautkosmetische Wirkstoffe, Pflanzenextrakte, Proteinhydrolysate, Puffersubstanzen, Komplexbildner und andere bekannte Hilfs- und Zusatzstoffe enthalten sein, wie sie in Haarshampoos, Badezusätzen, Duschbadpräparaten, flüssigen Seifen, flüssigen Hautreinigungsmitteln, aber auch in flüssigen Wasch- und Geschirrspülmitteln sowie flüssigen Haushaltsreinigungsmitteln üblich sind.

Von besonders hohem anwendungstechnischen Interesse ist die Viskositätserhöhung von wäßrigen Lösungen der genannten Art mit vergleichsweise geringem Tensidgehalt. In diesen Fällen verbessert der Zusatz der erfindungsgemäßen Ester in synergistischer Weise die Verdickbarkeit mit anorganischen Elektrolytsalzen.

Als anorganische Elektrolytsalze eignen sich alle wasserlöslichen Alkali-, Ammonium- und Erdalkalisalze, z. B. die Fluoride, Chloride, Bromide, Sulfate, Phosphate, Nitrate, soweit sie bei 20°C in einer Menge von wenigstens 1 Gew.-% in Wasser löslich sind. Bevorzugt werden die Chloride oder Sulfate eines Alkalimetalls, des Ammoniums oder Magnesiums verwendet. Besonders bevorzugt sind Natriumchlorid und Magnesiumchlorid. Die anorganischen Elektrolytsalze können den wäßrigen Tensidlösungen in Konzentrationen von 0,1 bis 5, vorzugsweise 0,1 bis 2 Gew.-% - bezogen auf die wäßrige Lösung - zugesetzt werden.

Die folgenden Beispiele sollen den Gegenstand der Erfindung näher erläutern, ohne ihn darauf einzuschränken.

### Beispiele

### I. Herstellungbeispiele:

### Glycerin-110 EO-tripalmitat/stearat (H1)

### a) Ethoxylierung:

In einem 5-l-Stahlautoklaven wurden
46,0 g (0,5 mol) Glycerin und
1,6 g (0,03 mol) Kaliumhydroxid
in Form einer 50 gew.-%igen wäßrigen Lösung vorgelegt. Anschließend wurde der Druckreaktor über einen Zeitraum von 1 h auf eine Temperatur von 110°C erhitzt und dabei evakuiert 30 mbar. Danach wurde die Reaktionstemperatur auf 160°C gesteigert und kontinuierlich über einen Zeitraum von 2 h 2420 g (55 mol) Ethylenoxid aufgepreßt, wobei sich ein autogener Druck von 4 bis 5 bar einstellte. Nach Beendigung der Ethylenoxidzugabe ließ man weitere 30 min nachreagieren; anschließend wurde die Reaktionsmischung abgekühlt und entspannt.

### b) Veresterung:

In einem 2-l-Dreihalskolben mit Rührer und Destillationsaufsatz wurden
1000 g (0,15 mol) Glycerin-110 EO-Adduktes (aus a),
126 g (0,47 mol) Talgfettsäure (C₁₆:C₁₈ = 1 : 1) und
3 g (0,02 mol) Methansulfonsäure
in Form einer 70 gew.-%igen wäßrigen Lösung vorgelegt und auf 90°C erwärmt. Danach wurde die Reaktionsmischung auf 160°C erhitzt und bei dieser Temperatur zunächst über einen Zeitraum von 4 h bei einem vermindertem Druck von 30 mbar und anschließend für weitere 12 h bei ca. 2 mm Hg gerührt, wobei das Kondensationswasser laufend aus dem Gleichgewicht abdestilliert wurde.

### Glycerin-130 EO-tripalmitat/stearat (H2)

Herstellbeispiel H1 wurde wiederholt, die Ethoxylierung jedoch mit 2860 g (65 mol) Ethylenoxid durchgeführt.

### Glycerin-150 EO-tripalmitat/stearat (H3)

Herstellbeispiel H1 wurde wiederholt, die Ethoxylierung jedoch mit 3300 g (75 mol) Ethylenoxid durchgeführt.

### II. Anwendungstechnische Beispiele

Die verdickende Wirkung der erfindungsgemäßen Ester wurde in wäßrigen Lösungen von Fettalkoholethersulfat-Salzen sowohl mit als auch ohne Zusatz von anorganischen Elektrolytsalzen untersucht. Die Viskositätsmessungen wurden in einem Brookfield RVT-Viskosimeter (Spindel 2-7, 10 Upm) durchgeführt. Der Feststoffgehalt der Lösung betrug 10 Gew.-%, die Temperatur 20°C. Die Ergebnisse sind in Tab.l zusammengefaßt.

### Rezeptur A:

| | |
|---|---|
| 37 g | Lauryalkohol-2,35 EO-sulfat-Na-Salz (Texapon^{(R)} PN-235, Fa.Pulcra S.A, Spanien) |
| 2 g | Glycerin-110 EO-tripalmitat/stearat (H1) |
| 61 g | Wasser |

### Rezeptur B:

| | |
|---|---|
| 37 g | Lauryalkohol-2,35 EO-sulfat-Na-Salz (Texapon^{(R)} PN-235, Fa.Pulcra S.A, Spanien) |
| 2 g | Glycerin-130 EO-tripalmitat/stearat (H2) |
| 61 g | Wasser |

### Rezeptur C:

| | |
|---|---|
| 37 g | Lauryalkohol-2,35 EO-sulfat-Na-Salz (Texapon^{(R)} PN-235, Fa.Pulcra S.A, Spanien) |
| 2 g | Glycerin-150 EO-tripalmitat/stearat (H3) |
| 61 g | Wasser |

### Rezeptur D:

| | |
|---|---|
| 37 g | Lauryalkohol-2,35 EO-sulfat-Na-Salz (Texapon^{(R)} PN-235, Fa.Pulcra S.A, Spanien) |
| 2 g | Polyethylenglycol-distereat Molgewicht des Polyethylenglycols: ca. 6000 (Eumulgin EO-33, Fa.Pulcra S.A., Spanien) |
| 61 g | Wasser |

**Tab. 1:**

| Viskositätsmessungen Angaben in 1000 cPs | | | | | | | | |
|---|---|---|---|---|---|---|---|---|
| Bsp. | Rezeptur | Zusatz von Natriumchlorid (Gew.-%) | | | | | | |
| | | 0 | 0,5 | 1,0 | 1,5 | 2,0 | 2,5 | 3,0 |
| 1 | A | 15 | 22 | 42 | 57 | 79 | | 118 |
| 2 | B | 20 | 30 | 48 | 61 | 80 | | 140 |
| 3 | C | 24 | 40 | 52 | 64 | 82 | | 160 |
| V1 | D | | | 1 | 9 | 38 | 82 | |

## Patentansprüche

1. Ester von Fettsäuren mit ethoxylierten Polyolen, dadurch erhältlich, daß man
a) Polyole in Gegenwart basischer Katalysatoren bei erhöhten Temperaturen mit 110 bis 200 mol Ethylenoxid pro mol Polyol ethoxyliert und
b) das Reaktionsprodukt anschließend in Gegenwart saurer Katalysatoren mit 1 bis 1,3 mol Fettsäure pro mol der im ursprünglichen Polyol enthaltenen Hydroxylgruppen umsetzt.

2. Ester nach Anspruch 1, **dadurch gekennzeichnet,** daß die Polyole ausgewählt sind aus der Gruppe, die von Glycerin, Diglycerin, Triglycerin, Oligoglycerinen mit einem durchschnittlichen Kondensationsgrad von 4 bis 10, Trimethylolpropan und Pentaerythrit gebildet wird.

3. Ester nach den Ansprüchen 1 und 2, **dadurch gekennzeichnet,** daß die Fettsäuren der Formel (I) folgen,
R¹CO-OH (I)
in der R¹CO für einen linearen oder verzweigten, aliphatischen, gegebenenfalls hydroxysubstituierten Acylrest mit 6 bis 22 Kohlenstoffatomen und 0, 1 2 oder 3 Doppelbindungen steht.

4. Verfahren zur Herstellung von Estern von Fettsäuren mit ethoxylierten Polyolen, **dadurch gekennzeichnet,** daß man
a) Polyole in Gegenwart basischer Katalysatoren bei erhöhten Temperaturen mit 110 bis 200 mol Ethylenoxid pro mol Polyol ethoxyliert und
b) das Reaktionsprodukt anschließend in Gegenwart saurer Katalysatoren mit 1 bis 1,3 mol Fettsäure pro mol der im ursprünglichen Polyol enthaltenen Hydroxylgruppen umsetzt.

5. Verfahren nach Anspruch 4, **dadurch gekennzeichnet,** daß man Polyole einsetzt, die ausgewählt sind aus der Gruppe, die von Glycerin, Diglycerin, Triglycerin, Oligoglycerinen mit einem durchschnittlichen Kondensationsgrad von 4 bis 10, Trimethylolpropan und Pentaerythrit gebildet wird.

6. Verfahren nach den Ansprüchen 4 und 5, **dadurch gekennzeichnet,** daß man basische Katalysatoren in die Ethoxylierung einsetzt, die ausgewählt sind aus der Gruppe, die von Natriumhydroxid, Kaliumhydroxid, Natriummethylat, calciniertem Hydrotalcit und mit Fettsäuren hydrophobiertem Hydrotalcit gebildet wird.

7. Verfahren nach mindestens einem der Ansprüche 4 bis 6, **dadurch gekennzeichnet,** daß man die Ethoxylierung bei einer Temperatur von 120 bis 190°C durchführt.

8. Verfahren nach mindestens einem der Ansprüche 4 bis 7, **dadurch gekennzeichnet,** daß man die Ethoxylierung bei einem Druck von 1 bis 5 bar durchführt.

9. Verfahren nach mindestens einem der Ansprüche 4 bis 8, **dadurch gekennzeichnet,** daß man Fettsäuren der Formel (I) einsetzt,
R¹CO-OH (I)
in der R¹CO für einen linearen oder verzweigten, aliphatischen, gegebenenfalls hydroxysubstituierten Acylrest mit 6 bis 22 Kohlenstoffatomen und 0, 1 2 oder 3 Doppelbindungen steht.

10. Verfahren nach mindestens einem der Ansprüche 4 bis 9, **dadurch gekennzeichnet,** daß man saure Katalysatoren in die Veresterung einsetzt, die ausgewählt sind aus der Gruppe, die von Methansulfonsäure, Butansulfonsäure, p-Toluolsulfonsäure, Naphthalinsulfonsäure, Alkylbenzolsulfonsäure und Sulfobernsteinsäure gebildet wird.

11. Verfahren nach mindestens einem der Ansprüche 4 bis 10, **dadurch gekennzeichnet,** daß man die Veresterung bei Temperaturen von 140 bis 200°C durchführt.

12. Verwendung der Ester nach den Ansprüchen 1 bis 3 als Verdikkungsmittel für wäßrige Tensidlösungen.

13. Verwendung der Ester nach dem Verfahren nach den Ansprüchen 4 bis 11 als Verdickungsmittel für wäßrige Tensidlösungen.

## Claims

1. Esters of fatty acids with ethoxylated polyols obtainable by
a) ethoxylating polyols with 110 to 200 moles of ethylene oxide per mole of polyol at elevated temperatures in the presence of basic catalysts and
b) subsequently reacting the reaction product with 1 to 1.3 moles of fatty acid per mole of the hydroxyl groups present in the original polyol in the presence of acidic catalysts.

2. Esters as claimed in claim 1, **characterized in that** the polyols are selected from the group consisting of glycerol, diglycerol, triglycerol, oligoglycerols with an average degree of condensation of 4 to 10, trimethylol propane and pentaerythritol.

3. Esters as claimed in claims 1 and 2, **characterized in that** the fatty acids correspond to formula (I):
R¹CO-OH (I)
in which R¹CO is a linear or branched, aliphatic, optionally hydroxy-substituted acyl radical containing 6 to 22 carbon atoms and 0, 1, 2 or 3 double bonds.

4. A process for the production of esters of fatty acids with ethoxylated polyols, **characterized in that**
a) polyols are ethoxylated with 110 to 200 moles of ethylene oxide per mole of polyol at elevated temperatures in the presence of basic catalysts and
b) the reaction product is subsequently reacted with 1 to 1.3 moles of fatty acid per mole of the hydroxyl groups present in the original polyol in the presence of acidic catalysts.

5. A process as claimed in claim 4, **characterized in that** the polyols used are selected from the group consisting of glycerol, diglycerol, triglycerol, oligoglycerols with an average degree of condensation of 4 to 10, trimethylol propane and pentaerythritol.

6. A process as claimed in claims 4 and 5, **characterized in that** basic catalysts selected from the group consisting of sodium hydroxide, potassium hydroxide, sodium methylate, calcined hydrotalcite and hydrotalcite hydrophobicized with fatty acids are used for the ethoxylation.

7. A process as claimed in at least one of claims 4 to 4, **characterized in that** the ethoxylation is carried out at a temperature of 120 to 190°C.

8. A process as claimed in at least one of claims 4 to 7, characterized in that the ethoxylation is carried out under a pressure of 1 to 5 bar.

9. A process as claimed in at least one of claims 4 to 8, **characterized in that** fatty acids corresponding to formula (I):
R¹CO-OH (I)
in which R¹CO is a linear or branched, aliphatic, optionally hydroxy-substituted acyl radical containing 6 to 22 carbon atoms and 0, 1, 2 or 3 double bonds, are used.

10. A process as claimed in at least one of claims 4 to 9, **characterized in that** acidic catalysts selected from the group consisting of methanesulfonic acid, butanesulfonic acid, p-toluenesulfonic acid, naphthalenesulfonic acid, alkyl benzenesulfonic acid and sulfosuccinic acid are used for the esterification.

11. A process as claimed in at least one of claims 4 to 10, **characterized in that** the esterification is carried out at temperatures of 140 to 200°C.

12. The use of the esters claimed in claims 1 to 3 as thickeners for aqueous surfactant solutions.

13. The use of the esters produced by the process claimed in claims 4 to 11 as thickeners for aqueous surfactant solutions.

## Revendications

1. Esters d'acides gras et de polyols éthoxylé qu'on peut obtenir en ce que :
a) on éthoxyle des polyols en présence de catalyseurs basiques à températures élevées avec 110 à 200 mol d'oxyde d'éthylène par mol de polyol et,
b) on fait réagir ensuite le produit de réaction en présence de catalyseurs acides avec 1 à 1,3 mol d'acide gras par mol de groupes hydroxyle contenus dans le polyol initial.

2. Esters selon la revendication 1, caractérisés en ce qu'on choisit les polyols dans le groupe constitué par la glycérine, la diglycérine, la triglycérine, les oligoglycérines ayant un taux moyen de condensation de 4 à 10, le triméthylolpropane et le pentaérythritol.

3. Esters selon les revendications 1 et 2, caractérisés en ce que les acides gras répondent à la formule (I),
R¹CO-OH
où R¹CO représente un radical acyle aliphatique, linéaire ou ramifié, le cas échéant hydroxysubstitué de 6 à 22 atomes de carbone et 0,1,2 ou 3 doubles liaisons.

4. Procédé de préparation d'esters d'acides gras et de polyols éthoxylés caractérisé en ce que :
a) on éthoxyle des polyols en présence de catalyseurs basiques à températures élevées avec 110 à 200 mol d'oxyde d'éthylène par mol de polyol et,
b) on fait réagir ensuite le produit de réaction en présence de catalyseurs acides avec 1 à 1,3 mol d'acide gras par mol de groupes hydroxyle contenus dans le polyol initial,

5. Procédé selon la revendication 4, caractérisé en ce qu'on utilise des polyols, qui sont choisis dans le groupe constitué par la glycérine, la diglycérine, la triglycérine, les oligoglycérines ayant un taux moyen de condensation de 4 à 10, le triméthylolpropane et le pentaérythritol.

6. Procédé selon les revendications 4 et 5, caractérisé en ce qu'on utilise des catalyseurs basiques pour l'éthoxylation, qu'on choisit dans le groupe formé par l'hydroxyde de sodium, l'hydroxyde de potassium, l'éthylate de sodium, l'hydrotalcite calcinée et l'hydrotalcite hydrophobée aux acides gras.

7. Procédé selon au moins l'une des revendications 4 à 6, caractérisé en ce qu'on réalise l'éthoxylation à une température de 120 à 190°C.

8. Procédé selon au moins l'une des revendications 4 à 7, caractérisé en ce qu'on réalise l'éthoxylation sous une pression de 1 à 5 bars.

9. Procédé selon au moins l'une des revendications 4 à 8, caractérisé en ce qu'on utilise des acides gras de formule (I) :
R¹CO-OH (I)
où R¹CO représente un radical acyle aliphatique, linéaire ou ramifié, le cas échéant hydroxysubstitué de 6 à 22 atomes de carbone et 0,1,2 ou 3 doubles liaisons.

10. Procédé selon au moins l'une des revendications 4 à 9, caractérisé en ce qu'on utilise des catalyseurs acides dans l'estérification, qu'on choisit dans le groupe qui est formé par les acides méthanesulfonique, butanesulfonique, p-toluènesulfonique, napthtalènesulfonique, alkylbenzènésulfonique et sulfosuccinique.

11. Procédé selon au moins l'une des revendications 4 à 10, caractérisé en ce qu'on réalise l'estérification à des températures de 140 à 200°C.

12. Utilisation des esters selon les revendications 1 à 3, comme épaississants de solutions aqueuses de tensioactifs.

13. Utilisation des esters selon les revendications 4 à 11, comme épaississant de solutions aqueuses de tensioactifs.
